# EUROPEAN PATENT APPLICATION

(11) **EP 4 667 041 A1**
(43) Date of publication of application: **24.12.2025**
(21) Application number: 24756664.9
(22) Date of filing: 31.01.2024
(51) Int. Cl.: A61M 39/10, A61M 1/36

(54) **LOCKING CONNECTOR**

(30) Priority: 17.02.2023 JP 2023023188
(71) Applicant: Nikkiso Co., Ltd., Tokyo 150-6022 (JP)
(72) Inventor: ISHIZAKI, Fumihiko, Higashimurayama-shi, Tokyo 189-8520 (JP); KAWAJIRI, Hiroyuki, Higashimurayama-shi, Tokyo 189-8520 (JP)
(74) Representative: Grosse Schumacher Knauer von Hirschhausen
(86) International application number: PCT/JP2024/003074
(87) International publication number: WO 2024/171813

(57) **Abstract**

A locking connector 1 comprises: a female connector 2 having a male thread portion 23 on the outer circumferential surface thereof; a male connector 3; a lock ring 4 that has a ring shape for surrounding the male connector 3 and pushes the male connector 3 toward the female connector 2 to lock the male connector 3 in a connected state through a female thread portion 41 being screwed onto the male thread portion 23, the female thread portion 41 being provided on the inner circumferential surface of the lock ring 4; one or more projected portions 5 formed on one of the outer circumferential surface of the male connector 3 and the inner circumferential surface of the lock ring 4; and a slope portion 6 formed on the other of the outer circumferential surface of the male connector 3 and the inner circumferential surface of the lock ring 4, the slope portion 6 having one or more inclined surfaces 61 formed at an angle with respect to the circumferential direction so that the diameter of the slope portion 6 gradually increases along the rotation direction of the screwing. The locking connector 1 is configured to prevent the lock ring 4 from loosening through the projected portions 5 coming into interference with the inclined surfaces 61 as the female thread portion 41 is screwed onto the male thread portion 23.

## Description

### TECHNICAL FIELD

The present invention relates to a locking connector.

### BACKGROUND OF THE INVENTION

Locking connectors called shunt connector are used for blood circuits in blood purification devices. A locking connector is used, e.g., to connect a tube extending from an artery-side puncture needle or a vein-side puncture needle to a tube constituting a blood circuit. The locking connector has a male connector provided at a tip of one of the tubes to be connected, a female connector provided at a tip of the other tube, and a lock ring that locks the male connector and the female connector in a connected state.

A conventional locking connector is known, which is configured such that a lock ring as a locking means is attached to a male connector, a male thread portion is formed on the outer circumferential surface of a female connector and a female thread portion to be screwed onto the male thread portion is formed on the inner circumferential surface of the lock ring, and by screwing the female thread portion onto the male thread portion, the connected state of the male connector and the female connector is locked while pushing the male connector against the female connector by the rock ring (see, e.g., Patent Literature 1).

### CITATION LIST

### PATENT LITERATURES

Patent Literature 1: JP 2017-077385A

### SUMMARY OF THE INVENTION

Components constituting the locking connector as described above, such as male connector, female connector, and lock ring, are resin components formed by molding a resin. The problem is that if burrs or sink marks (i.e., depressions) occur on these components due to molding defects, etc., it may not be possible to sufficiently suppress loosening of the lock ring. It may not be possible to sufficiently suppress the loosening of the lock ring also when scraping, etc. occurs on the circumferential surface of the lock ring.

Therefore, it is an object of the invention to provide a locking connector in which loosening of a lock ring can be suppressed.

A locking connector in an embodiment of the invention is a locking connector to connect ends of a pair of tubes to each other and comprises:
a first connector portion that is provided at an end of one of the tubes and comprises a male thread portion on an outer circumferential surface;
a second connector portion that is provided at an end of the other tube and is connected to the first connector portion;
a lock ring that is formed in an annular shape covering a periphery of the second connector portion, comprises, on an inner circumferential surface thereof, a female thread portion to be screwed onto the male thread portion and a movement restricting portion to restrict movement of the second connector portion toward a base end side, and by screwing the female thread portion onto the male thread portion, locks the first connector portion and the second connector portion in a connected state while pushing in the second connector portion toward the first connector portion by the movement restricting portion;
not less than one projecting portion formed on one of an outer circumferential surface of the second connector portion or the inner circumferential surface of the lock ring; and
a slope portion that is formed on the other of the outer circumferential surface of the second connector portion or the inner circumferential surface of the lock ring and
comprises not less than one inclined surface formed at an angle with respect to a circumferential direction about an axis of rotation of the screwing so that a diameter gradually increases along a direction of rotation of the screwing,
wherein as the female thread portion is screwed onto the male thread portion, the projecting portion rotates relative to the slope portion, the projecting portion interferes with the inclined surface with the rotation, and loosening of the lock ring is thereby prevented.

### Advantageous Effects of the Invention

According to the invention, it is possible to provide a locking connector in which loosening of a lock ring can be suppressed.

### BRIEF DESCRIPTION OF DRAWINGS

Fig. 1 is a diagram illustrating a blood circuit using locking connectors in an embodiment of the present invention.
Fig. 2 is an exploded perspective view showing the locking connector.
Fig. 3 is an exploded perspective view showing the locking connector.
Fig. 4 is a longitudinal cross-sectional view showing the locking connector in a connected state.
Fig. 5A is a perspective view showing a male connector.
Fig. 5B is a side view showing the male connector.
Fig. 5C is a front view showing the male connector.
Fig. 6A is a perspective view showing a rock ring.
Fig. 6B is a longitudinal cross-sectional view showing the rock ring.
Fig. 6C is a front view showing the rock ring.
Fig. 7 is an explanatory diagram illustrating prevention of loosening when fastening the lock ring.
Fig. 8A is a transverse cross-sectional view showing a modification of the locking connector.
Fig. 8B is a transverse cross-sectional view showing a modification of the locking connector.
Fig. 9 is a transverse cross-sectional view showing a modification of the locking connector.
Fig. 10 is a transverse cross-sectional view showing a modification of the locking connector.

### DETAILED DESCRIPTION OF THE INVENTION

### Embodiment

An embodiment of the invention will be described below in conjunction with the appended drawings.

### Blood circuit 100 using Locking connector 1

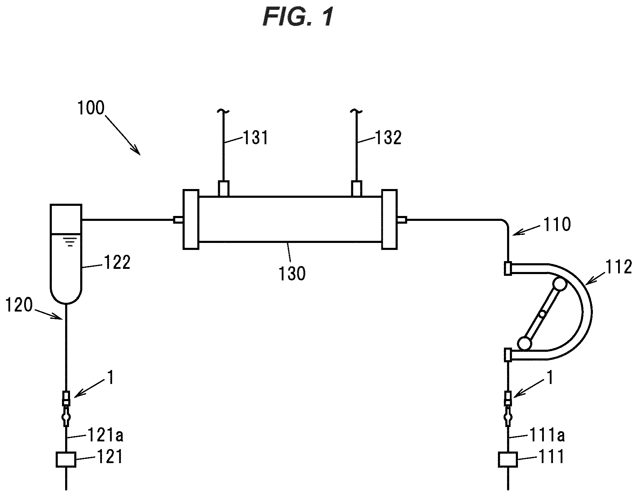

Fig. 1 is a diagram illustrating a blood circuit 100 using locking connectors 1 in the present embodiment. As shown in Fig. 1, the blood circuit 100 is used to extracorporeally circulate blood of a patient to perform blood purification, etc. during the course thereof for hemodialysis treatment, and is mainly composed of an artery-side blood circuit 110 with a tip connected to an artery-side puncture needle 111, and a vein-side blood circuit 120 with a tip connected to a vein-side puncture needle 121. Each of the artery-side blood circuit 110 and the vein-side blood circuit 120 is connected at a base end to a dialyzer 130 as a blood purifier. A dialysate introduction line 131 and a dialysate discharge line 132 are connected to the dialyzer 130, and through dialysis, unwanted substances (waste products) in the blood is removed into the dialysate inside the dialyzer 130.

Each of the artery-side blood circuit 110 and the vein-side blood circuit 120 is composed of a flexible tube. A peristaltic blood pump 112, which operates while squeezing the tube constituting the flow path of the artery-side side blood circuit 110, is placed at the middle of the artery-side side blood circuit 110. An air trap chamber 122 to remove bubbles is provided at the middle of the vein-side blood circuit 120. In this regard, the blood circuit 100 in Fig. 1 is merely an example and may include other components.

The locking connector 1 in the present embodiment is used, e.g., to connect the artery-side puncture needle 111 or the vein-side puncture needle 121 in the blood circuit 100. In the example shown in Fig. 1, the locking connector 1 is used to connect an end of a tube constituting the artery-side blood circuit 110 to an end of a tube 111a extending from the artery-side puncture needle 111. In the example shown in Fig. 1, another locking connector 1 is also used to connect an end of a tube constituting the vein-side blood circuit 120 to an end of a tube 121a extending from the vein-side puncture needle 121. However, it is not limited thereto. The locking connector 1 can be used to connect ends of tubes to each other, and the locking connector 1 can be used also at, e.g., a portion at which a replacement fluid line is connected to the vein-side blood circuit 120. Furthermore, the locking connector 1 can be also used for applications other than the blood circuit 100.

### Locking connector 1

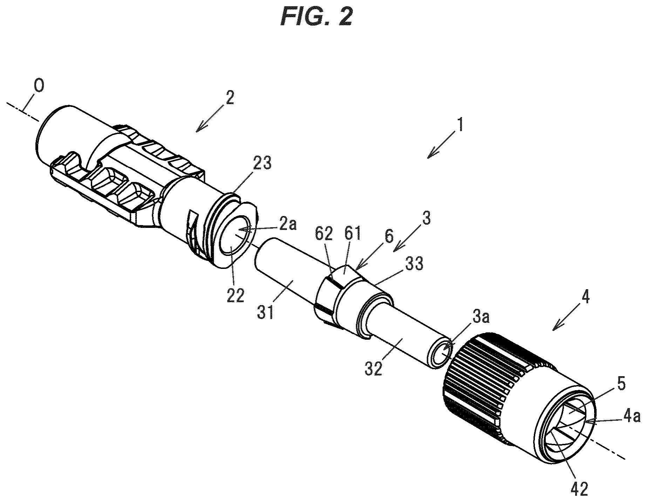

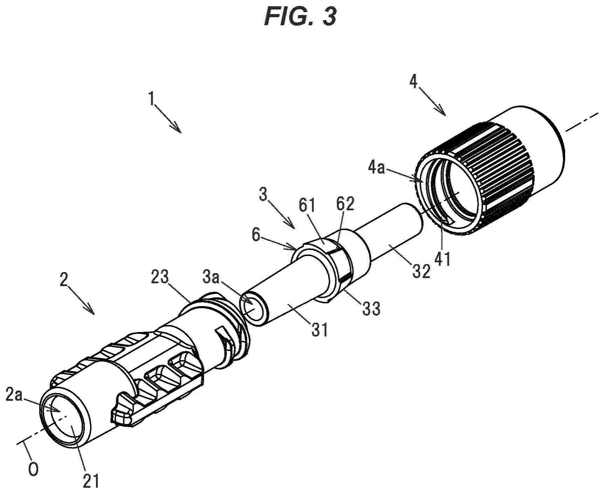

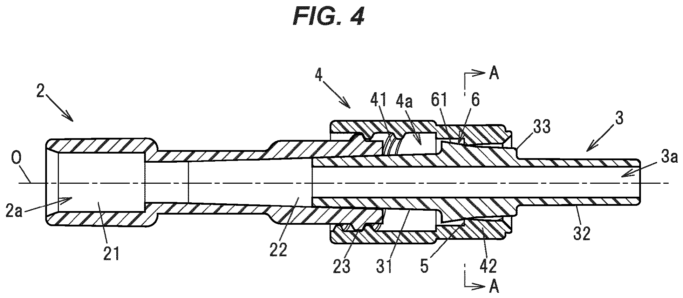

Figs. 2 and 3 are exploded perspective views showing the locking connector 1, and Fig. 4 is a longitudinal cross-sectional view showing the locking connector 1 in a connected state. As shown in Figs. 2 to 4, the locking connector 1 includes a female connector 2 provided at an end of one of tubes to be connected (not shown), a male connector 3 provided at an end of the other of the tubes to be connected (not shown), and a lock ring 4 to lock the female connector 2 and the male connector 3 in a connected state. The female connector 2 corresponds to the first connector portion of the invention, and the male connector 3 corresponds to the second connector portion of the invention. In this regard, however, the male-female relationship of the first connector portion and the second connector portion may be reversed. Hereinafter, a direction parallel to a rotation axis O of the lock ring 4 (i.e., an insertion direction of the male connector 3 into the female connector 2) will be referred to as the axial direction, and a rotation direction of the lock ring 4 (i.e., a rotation direction about the rotation axis O) will be referred to as the circumferential direction.

### Female connector 2

The female connector 2 is formed in a hollow cylindrical shape as a whole, and has a hollow portion 2a that penetrates the female connector 2 in the axial direction. A tube insertion portion 21, into which a tube (not shown) is inserted, is formed at one of openings of the hollow portion 2a, and a fitting portion 22, into which a tip portion of the male connector 3 (i.e., an insertion portion 31) is inserted and fitted, is formed at the other opening of the hollow portion 2a. The female connector 2 also has a male thread portion 23 provided on the outer circumferential surface thereof. The male thread portion 23 is provided on an end portion on the fitting portion 22 side.

The female connector 2 is a resin component which, in this example, is made of PC (polycarbonate). However, it is not limited thereto and the female connector 2 may be made of PP (polypropylene), high-density PE (polyethylene), thermoplastic elastomer, vinyl chloride, etc. Since the locking connector 1 may be subjected to thermal disinfection treatment (or sterilization treatment), a resin which is less likely to deform under heat is preferably used for the female connector 2.

### Male connector 3

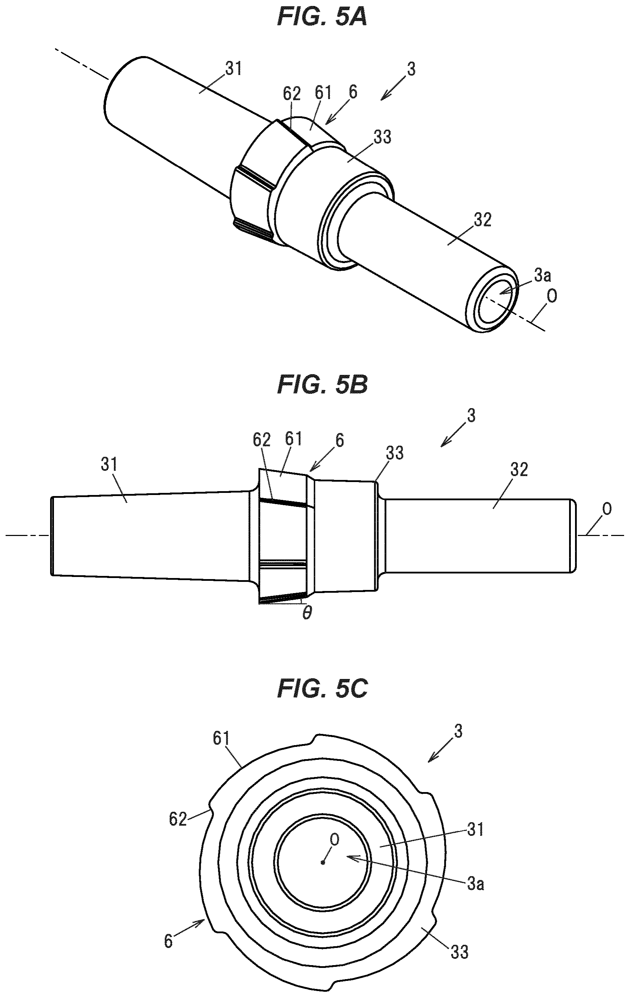

As shown in Figs. 5A to 5C, the male connector 3 is formed in a hollow cylindrical shape as a whole, and has a hollow portion 3a that penetrates the male connector 3 in the axial direction. The insertion portion 31 to be inserted into the fitting portion 22 of the female connector 2 is formed at a tip portion of the male connector 3, and a tube connection portion 32 to be inserted into a tube (not shown) is formed at a base end portion of the male connector 3. By inserting the insertion portion 31 of the male connector 3 into the fitting portion 22 of the female connector 2 and connecting the female connector 2 to the male connector 3, ends of the tubes are connected to each other through the hollow portions 2a and 3a, allowing, e.g., a liquid such as blood to flow. The insertion portion 31 is formed in a tapered shape with an outer diameter decreasing toward the tip to facilitate insertion into the fitting portion 22 and to make the insertion portion 31 less likely to come off when inserted into the fitting portion 22. The specific shape of the insertion portion 31 is specified by the ISO 80369-7 standard, and may be any shape which is in accordance with this standard.

The male connector 3 also has an interference portion 33 which is located between the insertion portion 31 and the tube connection portion 32 and is formed to have a larger outer diameter than the insertion portion 31 and the tube connection portion 32. The interference portion 33 is a portion that interferes with the lock ring 4 when fastening the lock ring 4 to restrict movement of the male connector 3 toward the base end side (toward right in Fig. 5B) and also prevent loosening of the lock ring 4. Details of the interference portion 33 will be described later.

The male connector 3 is a resin component which, in this example, is made of PC (polycarbonate) in the same manner as the female connector 2. However, it is not limited thereto and the male connector 3 may be made of PP (polypropylene), high-density PE (polyethylene), thermoplastic elastomer, vinyl chloride, etc. Since the locking connector 1 may be subjected to thermal disinfection treatment, a resin which is less likely to deform under heat is preferably used for the male connector 3.

### Lock ring 4

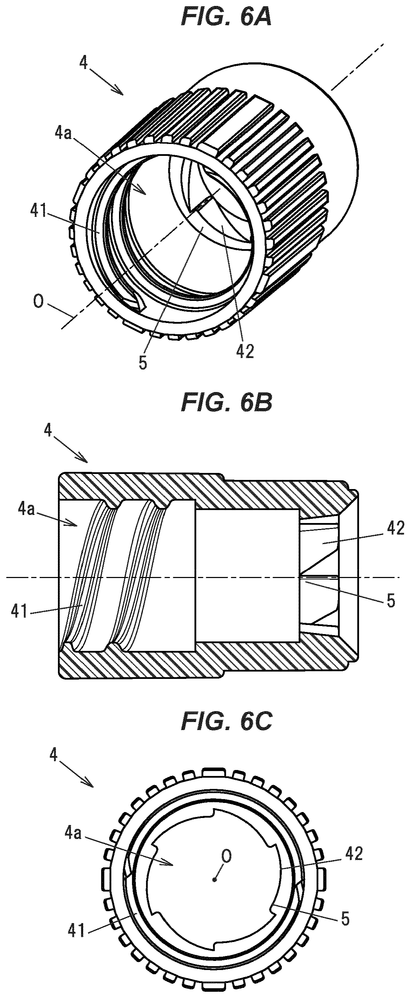

As shown in Figs. 6A to 6C, the lock ring 4 is formed in an annular shape covering the periphery of the male connector 3 (i.e., formed in a hollow cylindrical shape), and has a hollow portion 4a that penetrates the lock ring 4 in the axial direction. A female thread portion 41 to be screwed onto the male thread portion 23 of the female connector 2 is formed on an inner circumferential surface of the lock ring 4. The lock ring 4 also has a movement restricting portion 42 that is formed on the inner circumferential surface on the base end side relative to the female thread portion 41 and restricts movement of the male connector 3 toward the base end side by interfering with the interference portion 33 of the male connector 3. By screwing the female thread portion 41 onto the male thread portion 23, the lock ring 4 advances toward the female connector 2, and accordingly, the male connector 3 is pushed in toward the female connector 2 by the movement restricting portion 42, causing the female connector 2 and the male connector 3 to be locked in a connected state. In addition, in the present embodiment, the movement restricting portion 42, together with the interference portion 33 of the male connector 3, serves to prevent the loosening of the lock ring 4. Details of the movement restricting portion 42 will be described later.

The lock ring 4 is a resin component which, in this example, is made of PP (polypropylene) which is softer than the female connector 2 and the male connector 3. This makes a projecting portion 5 (described later) likely to deform, and it is thereby possible to further enhance the effect of preventing the loosening of the lock ring 4. However, it is not limited thereto and the lock ring 4 may be made of PC (polycarbonate), high-density PE (polyethylene), thermoplastic elastomer, vinyl chloride, etc. Since the locking connector 1 may be subjected to thermal disinfection treatment, a resin which is less likely to deform under heat is preferably used for the lock ring 4 in the same manner as the female connector 2 and the male connector 3 described above. The outer diameter of the lock ring 4 is, e.g., about 10 mm.

Description of Loosening prevention by Interference portion 33 and Movement restricting portion 42
In the present embodiment, it is configured to include not less than one projecting portion 5 which is formed on one of an outer circumferential surface of the male connector 3 or the inner circumferential surface of the lock ring 4, and a slope portion 6 which is formed on the other of the outer circumferential surface of the male connector 3 or the inner circumferential surface of the lock ring 4 and has not less than one inclined surface 61 formed at an angle with respect to the circumferential direction so that a diameter gradually increases along a direction of rotation of screwing the lock ring 4. Then, by screwing the female thread portion 41 of the lock ring 4 onto the male thread portion 23 of the female connector 2, the projecting portion 5 rotates relative to the slope portion 6, the projecting portion 5 interferes with the inclined surface 61 with the rotation, and the loosening of the lock ring 4 is thereby prevented. In this regard, "at an angle with respect to the circumferential direction" means being inclined so as to intersect with the circumferential direction.

In the present embodiment, the description will be given on the assumption that the projecting portion 5 is formed on the lock ring 4 and the slope portion 6 is formed on the male connector 3. In the present embodiment, the projecting portion 5 and the slope portion 6 are formed in substantially the same shape, which can also be interpreted as, e.g., having the slope portion 6 on the lock ring 4 and the projecting portion 5 on the male connector 3. The details will be described later.

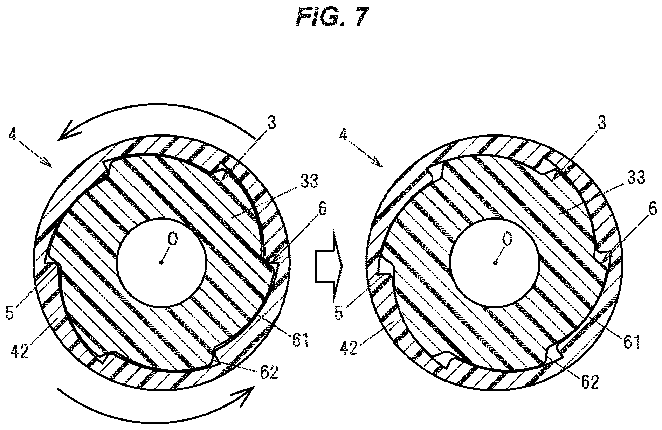

First, the slope portion 6 on the male connector 3 will be described. The slope portion 6 is formed on the outer circumferential surface of the interference portion 33 of the male connector 3. Fig. 7 is an explanatory diagram illustrating prevention of loosening when fastening the lock ring. Fig. 7 shows movement as viewed in a cross section taken along line A-A in Fig. 4. As shown in Fig. 7, the slope portion 6 has plural (six in this example) inclined surfaces 61 formed at an angle with respect to the circumferential direction so that the diameter gradually increases along the direction of rotation of screwing the lock ring 4 (the direction of the arrows in the drawing). Although the inclined surface 61 in the present embodiment is curved so as to be convex outward in the radial direction, the inclined surface 61 may be a flat surface which is not curved, or may be curved so as to be concave inward in the radial direction. Step portions 62 are formed between the inclined surfaces 61 adjacent to each other in the circumferential direction.

By having the slope portion 6, the projecting portions 5 formed on the inner circumferential surface of the lock ring 4 rotate relative to the slope portion 6 with the rotation of the lock ring 4 when rotated for fastening. Then, with the relative rotation, the projecting portions 5 are gradually pressed against the inclined surfaces 61 of the slope portion 6, resulting in a strong fastening state. This makes it possible to reliably prevent the loosening of the lock ring 4. In other words, in the present embodiment, the relative rotation between the lock ring 4 and the male connector 3 at the time of fastening of the lock ring 4 is used to cause the projecting portions 5 to climb on the inclined surfaces 61 of the slope portion 6, thereby preventing the loosening of the lock ring 4.

In this regard, if the angle of the inclined surface 61 is too steep, the projecting portions 5 may not be able to climb on the slope portion 6 and it may not be possible to sufficiently prevent the loosening of the lock ring 4. Therefore, the inclination angle of the inclined surface 61 is desirably relatively gentle. In more particular, when the number of inclined surfaces 61 is six as in the present embodiment, the height of the step portions 62 in the radial direction is preferably not less than 0.05 mm and not more than 2.0 mm. This is because molding is not stable when the step portions 62 are less than 0.05 mm, while the inclination angle of the inclined surfaces 61 becomes too large to provide a sufficient loosening prevention effect when the step portions 62 are more than 2.0 mm. The height of the step portions 62 in the radial direction is preferably not less than 0.1 mm and not more than 1.0 mm, more preferably not less than 0.2 mm and not more than 0.6 mm. In the present embodiment, the height of the step portions 62 in the radial direction is set to 0.4 mm. Since one inclined surface 61 occupies an angle range of about 60° in the present embodiment, the degree of increase in the radius from the inclined surface 61 to the rotation axis O should be not less than (0.05/60) mm and not more than (2.0/60) mm per 1°, preferably not less than (0.1/60) mm and not less than (1.0/60) mm, and more preferably not less than (0.2/60) mm and not more than (0.6/60) mm. The step portions 62 may be formed along a direction along the radial direction about the rotation axis O, or may be inclined with respect to the radial direction. In other words, the step portions 62 may be inclined with respect to the circumferential direction in the opposite direction to the inclined surface 61. In addition, the projecting portions 5 may be in contact with the step portions 62 in the fastened state in a case where, e.g., the lock ring 4 is rotated relative to the male connector 3 and is fastened at the moment the projecting portions 5 climb over the step portions 62. In such a case, by increasing the inclination angle of the step portions 62 with respect to the radial direction, the amount of interference between the projecting portions 5 and the step portions 62 can be increased to reliably prevent loosening.

Furthermore, as shown in Fig. 5B, the inclined surfaces 61 of the slope portion 6 in the present embodiment are formed at an angle with respect to the axial direction (the left-right direction in Fig. 5B) so that its diameter gradually decreases toward the base end side of the male connector 3 (toward right in Fig. 5B). As a result, the inclined surfaces 61 of the male connector 3 are gradually pushed into the movement restricting portion 42 (the projecting portions 5) as the lock ring 4 advances in the axial direction when fastening the lock ring 4, resulting in that a stronger fastening state is obtained. This allows for more reliable prevention of the loosening of the lock ring 4. An inclination angle θ of the inclined surfaces 61 with respect to the axial direction is preferably not less than 0.5° and not more than 45°. This is because it is difficult to stably mold when the inclination angle θ is less than 0.5°, while the inclined surfaces 61 may not fully enter the movement restricting portion 42 and the loosening prevention effect may not be obtained sufficiently when the inclination angle θ is more than 45°. In this regard, however, if the inclination angle θ is too small, the length of the inclined surfaces 61 in the axial direction needs to be very long. Therefore, the inclination angle θ is preferably not less than 1° and not more than 30°, more preferably not less than 2° and not more than 15°. In this example, the inclination angle θ is set to 6°, hence, the length of the slope portion 6 (the inclined surfaces 61) in the axial direction is about 6 mm.

Although the case where the inclined surfaces 61 are formed at an angle with respect to the axial direction has been described in the present embodiment, it is not limited thereto, and the projecting portions 5 may instead be formed at an angle respect to the axial direction (i.e., inclined so that the diameter gradually decreases toward the base end side (toward right in Fig. 4)). Moreover, both the inclined surfaces 61 and the projecting portions 5 may be formed at an angle with respect to the axial direction. By forming the inclined surfaces 61 or the projecting portions 5 at an angle with respect to the axial direction, it is possible to cause the inclined surfaces 61 and the projecting portions 5 to move relative to each other in the axial direction by fastening the lock ring 4 to a position where an appropriate fastening force is obtained, and it is possible to obtain a sufficient loosening prevention effect even when, e.g., burrs or sink marks, etc. occur on the inclined surfaces 61 or the projecting portions 5 due to molding defects, etc. or even when scraping occurs on the inclined surfaces 61 or the projecting portions 5.

Next, the projecting portions 5 formed on the lock ring 4 will be described. The projecting portions 5 are formed on the inner circumference of the movement restricting portion 42. The projecting portions 5 may be formed so as to extend toward the female thread portion 41 side beyond the movement restricting portion 42. In the present embodiment, the projecting portions 5 have an outline that is substantially transferred from the shape of the slope portion 6 when viewed in a cross section perpendicular to the axial direction, as shown in Figs. 6C and 7. That is, the projecting portions 5 are formed at an angle with respect to the circumferential direction so that the diameter gradually increases along the direction of rotation of screwing the lock ring 4. In addition, in the present embodiment, the number of projecting portions 5 and the number of inclined surfaces 61 are the same (six in this example), and the projecting portions 5 one-to-one correspond to the inclined surfaces 61. In other words, when viewed in a cross section perpendicular to the axial direction, the shape of the inner edge of the projecting portions 5 and the shape of the outer edge of the slope portion 6 are substantially similar to each other. This increases the contact area between the projecting portions 5 and the inclined surfaces 61, resulting in a stronger fastening state and a greater loosening prevention effect (see Fig. 7).

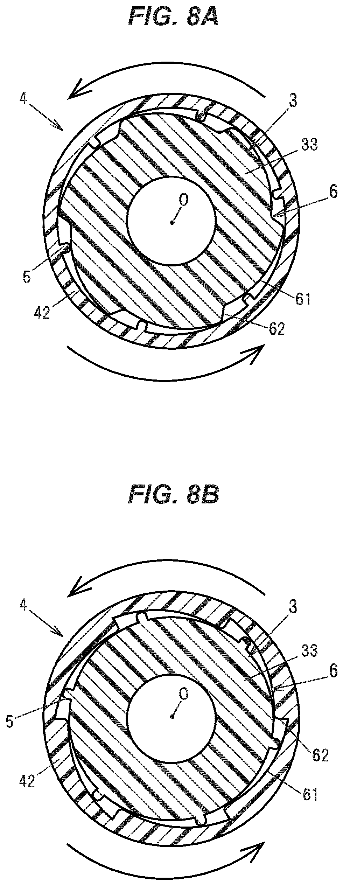

However, the shape of the projecting portions 5 is not limited thereto, and the projecting portions 5 may be, e.g., rib-shaped protrusions that protrude inward from the inner circumferential surface of the lock ring 4 as shown in Fig. 8A. In addition, although the case where the projecting portions 5 are provided on the lock ring 4 and the slope portion 6 is provided on the male connector 3 has been described in present embodiment, the same effect can be obtained also when the slope portion 6 is provided on the lock ring 4 and the projecting portions 5 are provided on the male connector 3 as shown in Fig. 8B.

### Functions and Effects of the embodiment

As described above, the locking connector 1 in the present embodiment includes not less than one projecting portion 5 formed on one of the outer circumferential surface of the male connector 3 or the inner circumferential surface of the lock ring 4, and the slope portion 6 that is formed on the other of the outer circumferential surface of the male connector 3 or the inner circumferential surface of the lock ring 4 and has not less than one inclined surface 61 formed at an angle with respect to the circumferential direction about the rotation axis O of screwing so that the diameter gradually increases along the direction of rotation of the screwing, and the locking connector 1 is configured such that by screwing the female thread portion 41 onto the male thread portion 23, the projecting portion 5 rotates relative to the slope portion 6, the projecting portion 5 interferes with the inclined surface 61 with the rotation, and the loosening of the lock ring 4 is thereby prevented.

Due to this configuration, it is possible to prevent the loosening of the lock ring 4 by using the rotation of the lock ring 4 during fastening, and it is possible to realize the locking connector 1 in which the loosening of the lock ring 4 is less likely to occur. In addition, the structure in the present embodiment is such that the projecting portions 5 climb up the inclined surfaces 61 due to relative rotation. Therefore, even if burrs or sink marks (i.e., depressions) occur due to molding defects, etc., the angle of the relative rotation changes accordingly in an appropriate manner, which allows a sufficient fastening force to be obtained and thus a sufficient loosening prevention effect to be obtained. For example, even if the dimension becomes small due to molding defects, the structure in which the projecting portions 5 climb up the inclined surfaces 61 allows the lock ring 4 to rotate to a position where a certain volume or area of interference is secured before stopping, hence, a stable fastening force can be obtained and a sufficient loosening prevention effect can be obtained. As a result, it is possible to suppress problems such that the connected portion using the locking connector 1 is unintentionally disconnected.

In addition, in the locking connector 1 of the present embodiment, at least one of the inclined surface 61 and the projecting portion 5 is formed at an angle with respect to the axial direction parallel to the rotation axis O so that the diameter gradually decreases toward the base end side. This makes it possible to use not only the rotation of the lock ring 4 during fastening but also the advancement of the lock ring 4 in the axial direction to prevent the loosening of the lock ring 4, and it is thereby possible to realize the locking connector 1 in which the loosening of the lock ring 4 is further less likely to occur.

### Modifications

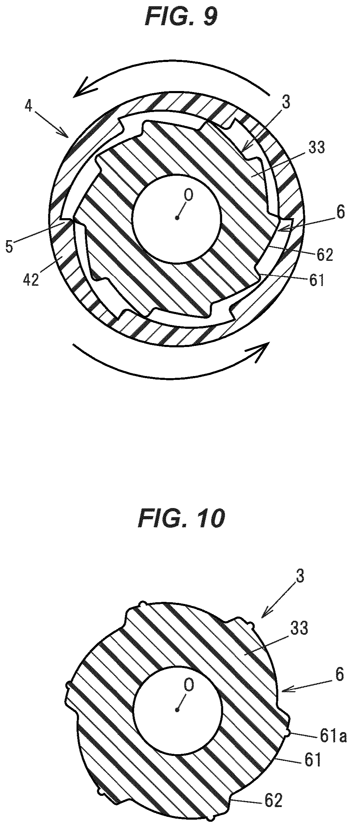

In the above embodiment, the case where the number of projecting portions 5 and the number of inclined surfaces 61 are the same has been described. In this case, however, the projecting portions 5 may climb over the step portions 62 all at once when fastening the lock ring 4 and the impact at the time of climbing over may cause an operator to feel a click-like tactile feedback. Providing a click-like tactile feedback can let the operator know that the fastening state is as intended, but feeling a click-like tactile feedback when the fastening is not yet sufficient could cause the operator to mistakenly think that the fastening is sufficient and to stop tightening the lock ring 4, resulting in insufficient fastening of the lock ring 4. Therefore, to suppress this click-like tactile feedback, the number of projecting portions 5 may be different from the number of inclined surfaces 61, as shown in Fig. 9. The number of projecting portions 5 is less than the number of inclined surfaces 61 in the example shown in the drawing, but the number of projecting portions 5 may be more than the number of inclined surfaces 61.

In addition, to further increase the fastening force and further enhance the loosening prevention effect, rib-shaped protrusions 61a may be further formed on the inclined surfaces 61, as shown in Fig. 10. By the rotation of the lock ring 4 during fastening, load is applied so that the protrusions 61a are pressed against the projecting portions 5, which further improves the loosening prevention effect.

### Summary of the embodiment

Technical ideas understood from the embodiment will be described below citing the reference signs, etc., used for the embodiment. However, each reference sign, etc., described below is not intended to limit the constituent elements in the claims to the members, etc., specifically described in the embodiment.
(1) A locking connector 1 to connect ends of a pair of tubes to each other, the locking connector 1 comprising: a first connector portion (female connector 2) that is provided at an end of one of the tubes and comprises a male thread portion 23 on an outer circumferential surface; a second connector portion (male connector 3) that is provided at an end of the other tube and is connected to the first connector portion 2; a lock ring 4 that is formed in an annular shape covering a periphery of the second connector portion 3, comprises, on an inner circumferential surface thereof, a female thread portion 41 to be screwed onto the male thread portion 23 and a movement restricting portion 42 to restrict movement of the second connector portion 3 toward a base end side, and by screwing the female thread portion 41 onto the male thread portion 23, locks the first connector portion 2 and the second connector portion 3 in a connected state while pushing in the second connector portion 3 toward the first connector portion 2 by the movement restricting portion 42; not less than one projecting portion 5 formed on one of an outer circumferential surface of the second connector portion 3 or the inner circumferential surface of the lock ring 4; and a slope portion 6 that is formed on the other of the outer circumferential surface of the second connector portion 3 or the inner circumferential surface of the lock ring 4 and comprises not less than one inclined surface 61 formed at an angle with respect to a circumferential direction about an axis of rotation of the screwing so that a diameter gradually increases along a direction of rotation of the screwing, wherein as the female thread portion 41 is screwed onto the male thread portion 23, the projecting portion 5 rotates relative to the slope portion 6, the projecting portion 5 interferes with the inclined surface 61 with the rotation, and loosening of the lock ring 4 is thereby prevented. This makes it possible to suppress the loosening of the lock ring 4.
(2) The locking connector 1 as defined by (1), wherein at least one of the inclined surface 61 and the projecting portion 5 is formed at an angle with respect to an axial direction parallel to the axis of rotation so that a diameter gradually decreases toward the base end side. This makes it possible to further suppress the loosening of the lock ring 4.
(3) The locking connector 1 as defined by (1), wherein the projecting portion 5 is formed at an angle with respect to the circumferential direction so that the diameter gradually increases along a screwing direction of the female thread portion 41. This increases the contact area between the projecting portion 5 and the inclined surface 61, making it possible to further suppress the loosening of the lock ring 4.
(4) The locking connector 1 as defined by (3), wherein the number of the projecting portions 5 is the same as the number of the inclined surfaces 61, and the projecting portions 5 one-to-one correspond to the inclined surfaces 61. This increases the contact area between the projecting portions 5 and the inclined surfaces 61, making it possible to further suppress the loosening of the lock ring 4.
(5) The locking connector 1 as defined by (1), wherein the number of the projecting portions 5 is different from the number of the inclined surfaces 61. This makes it possible to suppress the click-like tactile feedback when rotating the lock ring 4.

Although the embodiment of the invention has been described, the invention according to claims is not to be limited to the embodiment described above. Further, please note that not all combinations of the features described in the embodiment are necessary to solve the problem of the invention. In addition, the invention can be appropriately modified and implemented without departing from the gist thereof.

### REFERENCE SIGNS LIST

1: locking connector, 2: female connector (first connector portion), 21: tube insertion portion, 22: fitting portion, 23: male thread portion, 3: male connector (second connector portion), 31: insertion portion, 32: tube connection portion, 33: interference portion, 4: lock ring, 41: female thread portion, 42: movement restricting portion, 5: projecting portion, 6: slope portion, 61: inclined surface, 62: step portion

## Claims

1. A locking connector to connect ends of a pair of tubes to each other, the locking connector comprising:
a first connector portion that is provided at an end of one of the tubes and comprises a male thread portion on an outer circumferential surface;
a second connector portion that is provided at an end of the other tube and is connected to the first connector portion;
a lock ring that is formed in an annular shape covering a periphery of the second connector portion, comprises, on an inner circumferential surface thereof, a female thread portion to be screwed onto the male thread portion and a movement restricting portion to restrict movement of the second connector portion toward a base end side, and by screwing the female thread portion onto the male thread portion, locks the first connector portion and the second connector portion in a connected state while pushing in the second connector portion toward the first connector portion by the movement restricting portion;
not less than one projecting portion formed on one of an outer circumferential surface of the second connector portion or an inner circumferential surface of the lock ring; and
a slope portion that is formed on the other of the outer circumferential surface of the second connector portion or the inner circumferential surface of the lock ring and comprises not less than one inclined surface formed at an angle with respect to a circumferential direction about an axis of rotation of the screwing so that a diameter gradually increases along a direction of rotation of the screwing,
wherein as the female thread portion is screwed onto the male thread portion, the projecting portion rotates relative to the slope portion, the projecting portion interferes with the inclined surface with the rotation, and loosening of the lock ring is thereby prevented.

2. The locking connector according to claim 1, wherein at least one of the inclined surface and the projecting portion is formed at an angle with respect to an axial direction parallel to the axis of rotation so that a diameter gradually decreases toward the base end side.

3. The locking connector according to claim 1, wherein the projecting portion is formed at an angle with respect to the circumferential direction so that the diameter gradually increases along a screwing direction of the female thread portion.

4. The locking connector according to claim 3, wherein the number of the projecting portions is the same as the number of the inclined surfaces, and the projecting portions one-to-one correspond to the inclined surfaces.

5. The locking connector according to claim 1, wherein the number of the projecting portions is different from the number of the inclined surfaces.
